(19) Europäisches Patentamt
European Patent Office
Office européen des brevets

(11) EP 0 755 926 B1

(12) # FASCICULE DE BREVET EUROPEEN

(45) Date de publication et mention de la délivrance du brevet:
**27.09.2000 Bulletin 2000/39**

(51) Int Cl.[7]: **C07D 233/54**

(21) Numéro de dépôt: **95401779.4**

(22) Date de dépôt: **27.07.1995**

(54) **Procédé de préparation d'acétals du formyl-2 imidazole**

Verfahren zur Herstellung von Acetaten des 2-Formylimidazols

Process for the preparation of acetals of 2-formylimidazoles

(84) Etats contractants désignés:
**AT BE CH DE DK ES FR GB IE IT LI LU MC NL SE**

(43) Date de publication de la demande:
**29.01.1997 Bulletin 1997/05**

(73) Titulaire: **Clariant (France) S.A.**
**92800 Puteaux (FR)**

(72) Inventeurs:
- **Schouteeten, Alain**
  **F-95460 Ezanville (FR)**
- **Christidis, Yani**
  **F-75019 Paris (FR)**

(74) Mandataire: **Rinuy, Santarelli**
**14, avenue de la Grande Armée,**
**B.P. 237**
**75822 Paris Cédex 17 (FR)**

(56) Documents cités:
**EP-A- 0 018 568**

- **JOURNAL OF MEDICINAL CHEMISTRY, vol.34, no.4, Avril 1991, WASHINGTON US pages 1368 - 1376 G.A. KOOLPE ET AL. 'Quartenary Salts of 2-[(Hydroxyimino)methyl]imidazole. 5. Structure-Activity Relationships for Side-Chain Nitro-, Sulfone-, Amino-, and Aminosulfonyl-Substituted Analogs for Therapy against Anticholinesterase Intoxication'**
- **JOURNAL OF ORGANIC CHEMISTRY., vol.47, no.11, 21 Mai 1982, EASTON US pages 2196 - 2199 J.P. DIRLAM ET AL. 'Synthesis of Some Dihydroxyphenyl 4,5-Dichloroimidazol-2-yl Ketones: Compounds Related to Pyoluteorin'**
- **HOUBEN-WEYL, METHODEN DER ORGANISCHEN CHEMIE, BAND E8C: 'Hetarene III/Teil 3' 1994 , GEORG THIEME VERLAG , STUTTGART * page 4, dernier alinéa - page 5 ***

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne un procédé de préparation d'acétals du formyl-2 imidazole.

Le formyl-2 imidazole est un produit largement décrit dont on connaît certains de ses acétals tels que notamment le diméthoxyméthyl-2 imidazole (Gary A. KOOLPE et al, J. Med. Chem., 1991, 34, 1368-1376 ; T.S. MANOHARAN et al, J. Org. Chem., 1988 53, 1107-1110), qui sont obtenus par acétalisation classique du groupement aldéhyde. Quant au formyl-2 imidazole, il peut être obtenu, soit par oxydation de l'hydroxyméthyl-2 imidazole, soit par formylation de l'imidazole dont la fonction NH est transitoirement substituée par un groupement protecteur, soit enfin par hydrolyse d'un de ses dérivés aminals US-A-3812189 ; K.L. KIRK, J. Org. Chem., 1978, 43, 4381-4383 ; L.A.M. BASTIAANSEN et al, J. Org. Chem., 1986, 51, 1891-1894). Le formyl-2 imidazole et ses acétals sont des matières premières intéressantes pour accéder à divers produits présentant de précieuses propriétés thérapeutiques ou phytosanitaires, il est donc bénéfique de pouvoir disposer d'un procédé permettant d'obtenir rapidement et économiquement le formyl-2 imidazole ou l'un de ses acétals en un seul stade à partir de matières premières peu onéreuses. Dans ce but, la demanderesse à découvert avec étonnement un procédé simple et rapide permettant la préparation en un seul stade, d'acétals du formyl-2 imidazole.

La présente invention a donc pour objet un procédé de préparation d'un produit de formule (I)

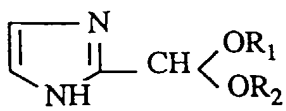

(I)

dans laquelle $R_1$ et $R_2$, soit identiques représentent un radical alcoyle en $C_1$-$C_4$, soit forment ensemble un groupement de formule (II)

$$-CHR_3-(CR_4R_5)_n-CHR_6- \qquad (II)$$

dans laquelle $R_3$, $R_4$, $R_5$ et $R_6$, identiques ou différents, représentent un atome d'hydrogène ou un radical alcoyle en $C_1$-$C_4$, et n représente 0 ou 1, caractérisé par le fait que l'on fait réagir un mélange aqueux de glyoxal et d'un éthanal de formule (III)

$$OHC-CH<\begin{matrix}OR_1\\OR_2\end{matrix} \qquad (III)$$

dans laquelle $R_1$ et $R_2$ ont la signification donnée ci-dessus, avec de l'ammoniac.

L'expression radical alcoyle en $C_1$-$C_4$ peut désigner, par exemple, un radical méthyle, éthyle, n-propyle, méthyl éthyle, n-butyle, méthyl-1 ou méthyl-2 propyle.

Les produits de formule (III) sont des produits qui peuvent être préparés par une des méthodes décrites dans les demandes de brevet européen n°249530 ou 316672, et certains sont des produits commerciaux.

Dans des conditions préférentielles de mise en oeuvre de l'invention, le procédé ci-dessus décrit est réalisé:

- en milieu aqueux,
- à une température inférieure à 60°C,
- avec un excès d'ammoniac et/ou de glyoxal.

Les exemples suivants illustrent l'invention sans toutefois la limiter.

## EXEMPLE 1

On chauffe à 40°C, sous agitation, 805 g d'une solution aqueuse contenant 153,3 g (9 moles) d'ammoniac,

puis dans cette solution agitée, maintenue à 45-50°C par un léger refroidissement extérieur, on introduit en 1 heure, 955 g d'une solution aqueuse contenant 209 g (3,6 moles) de glyoxal et 312,3 g (3 moles) de diméthoxy-2,2 éthanal. L'introduction terminée, la solution obtenue est chauffée 4 heures à 60°C, puis elle est concentrée sous pression réduite à 60°C à environ 900 g et elle est enfin refroidie à environ 15°C. Le produit attendu cristallise spontanément ; on l'isole par filtration puis on le recristallise par chaud et froid dans environ 500 g d'eau. On obtient ainsi 291,3 g (2,05 moles) de diméthoxyméthyl-2 imidazole cristallisé en prismes incolores, présentant un point de fusion de 122°C (littérature citée F = 120-121°C), RMN$^1$H ($CDCl_3$) $\delta$ 3,37 (s, 6H, $2CH_3$), 5,50 (s; 1H, CH), 7,06 (s,2H, aryl), 10,0 (s, 1H, NH).

**EXEMPLE 2**

On reproduit l'exemple 1 en n'utilisant que 3 moles de glyoxal au lieu de 3,6 moles. On obtient ainsi 251,6 g (1,77 mole) de diméthoxyméthyl-2 imidazole présentant un point de fusion de 122°C.

## Revendications

1. Procédé de préparation d'un produit de formule (I)

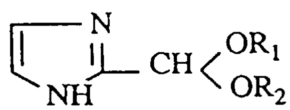

(I)

dans laquelle $R_1$ et $R_2$, soit identiques représentent un radical alcoyle en $C_1$-$C_4$, soit forment ensemble un groupement de formule (II)

$$-CHR_3\left(CR_4R_5\right)_n CHR_6- \qquad \text{(II)}$$

dans laquelle $R_3$, $R_4$, $R_5$ et $R_6$, identiques ou différents, représentent un atome d'hydrogène ou un radical alcoyle en $C_1$-$C_4$, et n représente 0 ou 1, caractérisé par le fait que l'on fait réagir un mélange aqueux de glyoxal et d'un éthanal de formule (III)

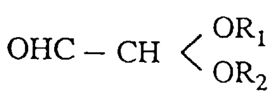

(III)

dans laquelle $R_1$ et $R_2$ ont la signification donnée ci-dessus avec de l'ammoniac.

## Patentansprüche

1. Verfahren zur Herstellung eines Produkts der Formel (I)

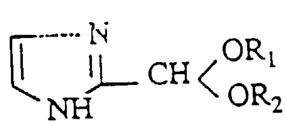

(I)

worin $R_1$ und $R_2$ entweder identisch sind und einen $C_1$-$C_4$-Alkylrest darstellen oder zusammen eine Gruppe der Formel (II) bilden

$$-CHR_3-(-CR_4R_5-)_n-CHR_6- \qquad (II)$$

worin $R_3$, $R_4$, $R_5$ und $R_6$ identisch oder verschieden sind, ein Wasserstoffatom oder einen $C_1$-$C_4$-Alkylrest darstellen und n 0 oder 1 darstellt, dadurch gekennzeichnet, dass man eine wässrige Mischung von Glyoxal und einem Ethanal der Formel (III)

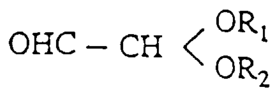

(III)

worin $R_1$ und $R_2$ die oben angegebene Bedeutung haben, mit Ammoniak umsetzt.

## Claims

**1.** Preparation process for a product of formula (I):

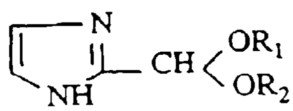

(I)

in which $R_1$ and $R_2$, either are identical and represent a $C_1$-$C_4$ alkyl radical, or together form a group of formula (II):

$$-CH_3-(-CR_4R_5-)_n-CHR_6- \qquad (II)$$

in which $R_3$, $R_4$, $R_5$ and $R_6$, identical or different, represent a hydrogen atom or a $C_1$-$C_4$ alkyl radical, and n represents 0 or 1, characterized in that an aqueous mixture of glyoxal and an ethanal of formula (III):

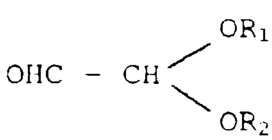

(III)

in which $R_1$ and $R_2$ have the meaning given above, is reacted with ammonia.